# FASCICULE DE BREVET EUROPEEN

(11) **EP 1 828 392 B1**
(45) Date de publication et mention de la délivrance du brevet: **16.06.2010**
(21) Numéro de dépôt: 05826569.5
(22) Date de dépôt: 13.12.2005
(51) Int. Cl.: C12P 7/40

(54) **PRODUCTION D' ACIDES DICARBOXYLIQUES PAR DES SOUCHES MUTANTES AMELIORÉES DE YARROWIA LIPOLYTICA**
HERSTELLUNG VON DICARBONSÄURE DURCH VERBESSERTE YARROWIA LIPOLYTICA-MUTANTENSTÄMME
PRODUCTION OF DICARBOXYLIC ACIDS BY IMPROVED MUTANT STRAINS OF YARROWIA LIPOLYTICA

(30) Priorité: 15.12.2004 FR 0413468
(43) Date de publication de la demande: 05.09.2007
(73) Titulaire: Institut Français du Pétrole, 92852 Rueil Malmaison Cedex (FR); Centre National de la Recherche Scientifique, 75794 Paris Cedex 16 (FR); INSTITUT NATIONAL DE LA RECHERCHE AGRONOMIQUE (INRA), 75338 Paris Cédex 07 (FR)
(72) Inventeur: NICAUD, Jean-Marc, F-78850 Trappes (FR); THEVENIEAU, France, M4 1PY MANCHESTER (GB); LE DALL, Marie-Thérèse, F-92230 Gennevilliers (FR); MARCHAL, Rémy, F-78400 Chatou (FR)
(86) Numéro de dépôt international: PCT/FR2005/003140
(87) Numéro de publication internationale: WO 2006/064131

(56) Documents cités:
- WO-A-03/100013
- US-A- 5 648 247
- WANG HUIJIE J ET AL: "Evaluation of acyl coenzyme A oxidase (Aox) isozyme function in the n-alkane-assimilating yeast Yarrowia lipolytica" JOURNAL OF BACTERIOLOGY, vol. 181, no. 17, septembre 1999 (1999-09), pages 5140-5148, XP002336152 ISSN: 0021-9193
- WACHE YVES ET AL: "Role of beta-oxidation enzymes in gamma-decalactone production by the yeast Yarrowia lipolytica" APPLIED AND ENVIRONMENTAL MICROBIOLOGY, vol. 67, no. 12, décembre 2001 (2001-12), pages 5700-5704, XP002336153 ISSN: 0099-2240
- WANG HUIJIE ET AL: "Cloning and characterization of the peroxisomal acyl CoA oxidase ACO3 gene from the alkane-utilizing yeast Yarrowia lipolytica" YEAST, vol. 14, no. 15, novembre 1998 (1998-11), pages 1373-1386, XP008049873 ISSN: 0749-503X
- FICKERS P ET AL: "New disruption cassettes for rapid gene disruption and marker rescue in the yeast Yarrowia lipolytica." JOURNAL OF MICROBIOLOGICAL METHODS, vol. 55, no. 3, décembre 2003 (2003-12), pages 727-737, XP002336154 ISSN: 0167-7012 cité dans la demande
- SMIT M.S. ET AL: '[alpha],[omega]-Dicarboxylic acid accumulation by acyl-CoA oxidase deficient mutants of Yarrowia lipolytica' BIOTECHNOLOGY LETTERS vol. 27, no. 12, 01 Juin 2005, pages 859 - 864, XP019230875

## Description

### Domaine de l'invention

L'invention concerne un procédé de production d'acides dicarboxyliques par une fermentation mettant en oeuvre une souche mutante de la levure *Yarrowia lipolytica* à partir d'un substrat de bioconversion.

Les acides dicarboxyliques (aussi appelés "diacides") sont utilisés comme matières premières par exemple dans la synthèse de polyamides et de polyesters, d'huiles lubrifiantes, d'agents plastifiants ou de parfums.

Les procédés de production des diacides varient suivant le nombre d'atomes de carbone du squelette carboné du diacide considéré (Johnson RW, Pollock CM, Cantrell RR, Editors Kirk-Othmer Encyclopedia of Chemical Technology, 4th Edition, 1983, pp. 118-136). Ainsi, l'acide azélaïque (diacide en C9) est obtenu classiquement par oxydation chimique de l'acide oléique par l'ozone tandis que l'acide sébacique (diacide en C10) est produit par oxydation alcaline de l'acide ricinoléique. L'acide dodécanedioïque (diacide en C12) est un produit de la pétrochimie. La voie microbiologique est utilisée pour la production d'acide brassylique (diacide en C13) à partir de tridécane.

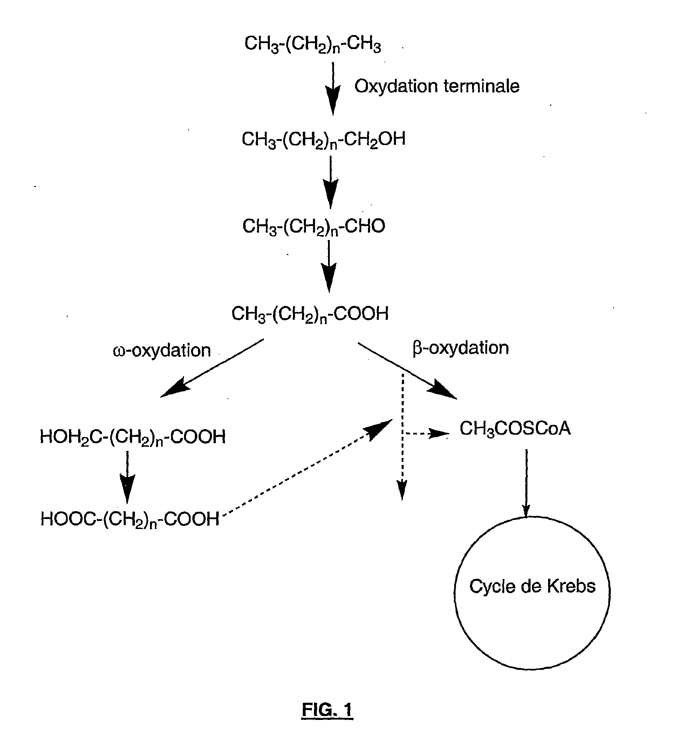

Compte tenu de la diversité des diacides qui sont utilisés dans les différentes applications, l'intérêt d'une voie de production applicable à la plus large gamme possible de diacides est incontestable. Bien que se caractérisant par une vitesse de réaction plus lente que celle de la voie chimique, la voie biologique présente l'intérêt d'être applicable à une grande variété de substrats (le processus de production des diacides par voie biologique est représenté schématiquement à la Figure 1).

En fait, de nombreuses espèces microbiennes sauvages telles que *Cryptococcus neoformans, Pseudomonas aeruginosa, Candida cloacae,* etc. sont capables d'excréter de faibles quantités de diacides (Chan et al.: Stimulation of n-alkane conversion to dicarboxylic acid by organic-solvent- and detergent-treated microbes. Appl. Microbiol. Biotechnol., 34, 1991, 772-777; Shiio et al.: Microbial Production of Long-chain Dicarboxylic Acids from n-Alkanes. Part I. Screening and Properties of Microorganisms Producing Dicarboxylic Acids. Agr. Biol. Chem. 35, No. 13, 1971, p. 2033-2042).

Cependant, pour obtenir des excrétions substantielles de diacides, il convient d'utiliser des mutants qui ont été bloqués au niveau de la β-oxydation. Pour de nombreuses espèces, de tels mutants ont été obtenus par une mutagénèse au hasard, suivie d'une sélection appropriée (brevet EP 0 229 252 B1 ; Shiio et al.; Jiao et al. Isolation and Enzyme Determination of Candida tropicalis Mutants for DCA Production. J. Gen. Appl. Microbiol. 2000, 46: 245-249).

Une voie beaucoup plus élégante mais plus contraignante, mettant en oeuvre les techniques de mutagénèse dirigée, a cependant été développée pour *Candida tropicalis.* À partir d'une souche sauvage appartenant à cette espèce, Picataggio et *al.* ont disrupté séquentiellement les quatre gènes codant les deux isoenzymes de l'acyl-CoA oxydase (Aox) qui catalysent la première étape de la β-oxydation (Determination of Candida tropicalis Acylcoenzyme A Oxidase Isoenzyme Function by Sequential Gene Disruption. Mol. Cell. Biol. 11, 1991, 4333-4339, et brevet US 5 254 466 A1).

Ces mêmes auteurs ont ensuite surexprimé les gènes codant pour la cytochrome P450 mono-oxygénase et la NADPH-cytochrome réductase, qui constituaient les activités limitant la cinétique de la conversion des *n*-alcanes en diacides (Picataggio et al.: Metabolic Engineering of Candida tropicalis for the Production of Long-chain Dicarboxylic Acids. Biotechnol. 10, 1992, 894-898, et brevet US 5 648 247 A1). Cependant, les mutants de *Candida tropicalis* produits selon un système d'amplification multicopies ne semblent pas totalement stables et se prêtent à des réversions possibles. C'est pour cette raison que des améliorations ont dû être apportées à l'art antérieur (demande de brevet US 200410014198 A1).

Whang et al. (J. of Bacteriology, vol.181, no17, septembre 1999 pages 5140-5148*)* décrit des mutants de *Yarrowia lipolytica* dont les gènes codant pour l'acyl-CoA oxydase ont été disruptés.

Wache et al. (Applied and Environmental Microbiology, vol.67, no 12, décembre 2001, pages 5700-5704) décrit la transformation du ricinoleate de méthyle en γ-decalactone, par des souches mutantes de *Yarrowia lipolytica* dans lesquelles des gènes codant pour l'acyl-CoA oxydase ont été disruptés.

### Objet de l'invention

L'objet de l'invention est de remédier aux inconvénients de l'art antérieur. On a en effet découvert qu'il était possible, de manière avantageuse, de produire des diacides en utilisant des mutants de *Yarrowia lipolytica* dont les gènes codant pour l'acyl-CoA oxydase ont été disruptés.

Les diacides que le procédé de l'invention vise à préparer sont des composés organiques à chaîne hydrocarbonée linéaire d'au moins 10 atomes de carbone comportant une fonction carboxylique à chaque extrémité de la chaîne.

On utilise comme microorganisme un mutant de *Yarrowia lipolytica* dans lequel au moins les gènes *POX2, POX3, POX4* et *POX5* ont été disruptés, pour le bloquer partiellement au niveau de la β-oxydation.

Hormis les acyl-CoA oxydases codées par les gènes *POX2, POX3, POX4* et *POX5,* deux autres acyl-CoA oxydases codées par les gènes *POX1* et *POX6,* de fonction inconnue, sont présentes dans le génome de *Yarrowia lipolytica.*

Ces deux acyl-CoA oxydases sont impliquées dans la reconsommation des diacides biosynthétisés par élimination séquentielle de deux atomes de carbone. La disruption supplémentaire des gènes *POX*1 et *POX*6 entraîne donc la production de diacides correspondant au profil du substrat de bioconversion. Par exemple, si on utilise l'huile de tournesol oléique, composée en majorité d'acides gras à 18 atomes de carbone, comme substrat de bioconversion, la souche délétée de l'ensemble des gènes *POX,* produira en majorité des diacides à 18 atomes de carbone.

D'autre part, le substrat de bioconversion peut être stocké sous forme de triglycérides au sein même de la cellule sous forme de corps lipidiques et devenir donc inaccessible pour sa bioconversion en diacides. Des gènes ont été identifiés, par analyse protéomique, comme étant impliqués dans l'accumulation du substrat de bioconversion. Nous avons identifié les gènes: *DGA*1 (acyl-CoA:diacylglycerol acyltransferase) *TGL*1 (triacylglycerol lipase), *G3P* (glycerol-3-phosphate dehydrogénase), *SCP2* (transporteur putatif de stérol), *LR*01 (lecithine cholesterole acyltransferase putatif) ainsi que les IFP 621 (fonction inconnue), IPF 905 (fonction inconnue), et IPF 2569 (sous-unité NADH-ubiquinone réductase).

La modification de l'activité de ces gènes (par disruption ou surexpression) entraîne donc une diminution de l'accumulation du substrat de bioconversion sous forme de corps lipidiques au sein de la cellule de *Yarrowia lipolytica.*

On notera que le système génétique de *Yarrowia lipolytica* est très différent de celui de *Candida tropicalis.* Contrairement à *Candida tropicalis* qui est une levure diploïde, *Yarrowia lipolytica* est en effet une espèce haploïde. Chez ce dernier microorganisme, les opérations de délétion génique sont donc beaucoup plus efficaces et plus sûres, du fait même de la présence d'un jeu unique de chromosomes.

D'autre part, un promoteur du gène POX2 codant pour l'acyl-CoA oxydase est utilisé pour surexprimer des gènes tels que, par exemple, ceux codant pour la cytochrome P450 mono-oxygénase et pour la NADPH-cytochrome réductase. Le promoteur pPOX2 a la propriété d'être un promoteur fort inductible par les substrats de bioconversion. Ainsi, la surexpression d'un gène d'intérêt est réalisée par l'addition d'une seule copie de gène sous le contrôle du promoteur pPOX2, permettant d'obtenir des mutants de *Yarrowia lipolytica* efficaces, stables et non-revertants contrairement aux mutants de *Candida tropicalis* obtenus par un système d'amplification multicopies.

Un autre avantage de *Yarrowia lipolytica* sur *Candida tropicalis* pour la production de diacides à partir d'esters d'acides gras ou d'huiles naturelles, par exemple végétales, est le suivant : la transformation des huiles naturelles en diacides par *Candida tropicalis* nécessite une hydrolyse chimique au moins partielle du substrat avant la mise en oeuvre de la fermentation (voir brevet US 5 962 285). Cette hydrolyse est réalisée par une saponification conduite en présence d'hydroxyde de calcium ou de magnésium. Elle produit les sels d'acides gras (savons) correspondants. Or, *Yarrowia lipolytica* présente la capacité d'assimiler les triglycérides comme source de carbone. La première étape de ce catabolisme implique l'hydrolyse des triglycérides en acides gras libres et glycérol par les enzymes lipolytiques (lipases), identifiées par Peters et Nelson en 1951. Une activité lipase extracellulaire et deux lipases membranaires de 39 et 44 kDa (Barth et al., Yarrowia lipolytica. in: Nonconventional Yeasts in Biotechnology A Handbook (Wolf, K., Ed.), Vol. 1, 1996, pp. 313-388. Springer-Verlag) furent ensuite décrites. *Yarrowia lipolytica* est capable de produire plusieurs lipases (activité extracellulaire, membranaire et intracellulaire). Récemment, les gènes correspondants aux lipases décrites ont été identifiés. Le gène *LIP*2 code pour une lipase extracellulaire, Lip2p (Pignede *et al.,* 2000). Il a été démontré qu'elle hydrolysait préférentiellement les triglycérides à longue chaîne des résidus oléiques (Barth *et al,* 1996).

*Yarrowia lipolytica* hydrolyse donc directement les esters et les huiles naturelles en acides gras libres et glycérol dans des conditions de pH compatibles avec la conduite de la fermentation. Dans ces conditions, l'hydrolyse de l'ester ou de l'huile et sa conversion en diacides ont lieu simultanément, ce qui présente l'avantage de mener à un protocole opératoire simplifié puisque l'étape d'hydrolyse chimique est éliminée.

### Description détaillée de l'invention

De manière plus détaillée, l'invention propose un procédé production d'au moins un acide dicarboxylique qui comprend :
(a) une phase de croissance, dans laquelle on met en culture une souche mutante de *Yarrowia lipolytica* disruptée au moins pour les gènes *POX2, POX3, POX4* et *POX5* (codant pour l'acyl-CoA oxydase) dans un milieu de culture constitué essentiellement d'un substrat énergétique qui comprend au moins une source de carbone et une source d'azote ;
(b) une phase de bioconversion, dans laquelle on soumet ladite souche à un substrat de bioconversion choisi parmi les *n*-alcanes d'au moins 10 atomes de carbone, les acides gras d'au moins 10 atomes de carbone, les esters d'alkyle de 1 à 4 atomes de carbone de ces acides gras, tels que les mélanges d'esters méthyliques ou éthyliques, ou encore les huiles naturelles (mélanges d'esters d'acides gras du glycérol), en présence d'un substrat énergétique ; et
(c) une phase de récupération de l'acide dicarboxylique formé.

Les souches utilisées dans le procédé de l'invention dérivent de la souche sauvage *Yarrowia lipolytica* W29 (ATCC 20460, répertoriée sous CLIB89 dans la Collection de Levures d'Intérêt Biotechnologique-CLIB).

Ainsi, on peut utiliser de nouvelles souches mutantes dérivant de la souche *Yarrowia lipolytica* ATCC 20460 par l'intermédiaire de la souche Po1d [souche auxotrophe pour la leucine (leu-) et l'uracile (ura-)], décrite dans la revue de G. Barth *et al..* Elle est répertoriée sous CLIB139 dans la CLIB.

L'obtention de ces nouvelles souches mutantes (MTLY37, MTLY66, MTLY74, MTLY79, MTLY80, MTLY81, FT 120 et FT 130) sera décrite plus loin.

Dans le procédé de production de diacides selon l'invention, on met la souche mutante choisie en culture dans un milieu constitué essentiellement d'un substrat énergétique qui comprend au moins une source de carbone et une source d'azote jusqu'à la fin de la croissance. On ajoute alors le substrat de bioconversion (alcane ou mélange d'alcanes, acide gras ou mélange d'acides gras, ester d'acide gras ou mélange d'esters d'acides gras ou huile naturelle ou mélange de ces différents substrats) de façon à initier la bioconversion en diacides et on récupère les diacides formés par une technique connue de l'homme du métier, telle que la précipitation des sels de calcium.

Lors de la phase de bioconversion, le milieu de culture peut comprendre un apport de substrat énergétique secondaire consistant en général en au moins un composé polyhydroxylé, tel que par exemple le glycérol ou un sucre.

### 1. Obtention de la souche mutante MTLY37

Pour obtenir cette souche, on peut procéder comme suit :
1) on séquence le gène d'intérêt (ou bien on dispose de la séquence de ce gène dans une banque de données) ;
2) on construit une cassette de disruption par PCR ("Polymerase Chain Reaction") ou par clonage, en utilisant un marqueur contre-sélectionnable, par exemple le marqueur URA3 (avec lequel on peut sélectionner pour le phénotype Ura+ ou pour le phénotype Ura-) ;
3) on sélectionne les souches avec le gène d'intérêt délété (transformation et sélection des transformants (avantageusement Ura+ si le marqueur est URA3) et on vérifie la disruption du gène.

On décrit ci-après une méthode particulière d'obtention de la souche mutante MTLY37.

On clone tout d'abord et on séquence des gènes *POX* de la souche sauvage, dont les séquences sont différentes de celles de *Candida tropicalis.* On construit ensuite des cassettes de disruption des gènes codant pour les iso-enzymes de l'acyl-CoA oxydase. On disrupte les gènes de l'acyl-CoA oxydase en utilisant le marqueur sélectionnable URA3. On amplifie les régions promotrices et terminatrices par une première PCR, en utilisant des paires d'oligonucléotides spécifiques, ce qui élimine la séquence complète du cadre de lecture ouverte (ORF : "Open Reading Frame"). On effectue ensuite une seconde PCR avec les amorces externes et les produits de PCR des promoteurs et terminateurs, qui fusionnent via une extension commune de 20 bp comportant un site pour l'enzyme de restriction I-Scel. Le produit de PCR est cloné pour donner une série de plasmides (désignés par pPOX-PT) contenant le module promoteur-terminateur (cassette de disruption 2).

Un gène URA3 est introduit dans le site I-Scel de la cassette POX-PT. Une série de plasmides pPOX-PUT contenant le module promoteur-URA3-terminateur est construite (cassette de disruption 1). On appelle ces constructions p*POX1*-PUT, p*POX2*-PUT, p*POX3*-PUT, p*POX4*-PUT. et p*POX5*-PUT pour les plasmides contenant la cassette de disruption 1, d'une part, et p*POX1*-PT, p*POX2*-PT, p*POX3*-PT, p*POX4*-PT, et p*POX5*-PT pour les plasmides contenant la cassette de disruption 2, d'autre part.

Les cassettes de disruption sont amplifiées par PCR avec les amorces spécifiques externes en utilisant par exemple la *Pfu* polymérase (fournie par Stratagene, La Jolla, Californie). L'analyse finale des séquences de protéines révèle que les acyl-CoA oxydases de *Yarrowia lipolytica* ont un degré d'identité de 45 % (50 % de similarité) avec celui des autres levures. Le degré d'identité entre elles varie de 55 à 70 % (65 à 76 % de similarité).

Après avoir construit les cassettes de disruption des gènes codant pour les acyl-CoA oxydase, on peut réaliser la transformation de *Yarrowia lipolytica* par différentes méthodes. On peut mettre en oeuvre une électroporation, dans laquelle l'ADN rentre grâce au choc électrique. Plus avantageusement, on mettra en oeuvre la méthode à l'acétate de lithium et au polyéthylène glycol. Cette méthode est décrite par Gaillardin et al.: LEU2 Directed Expression of Beta-gallactosidase Activity and Phleomycin Resistance in Yarrowia lipolytica. Curr. Genet. 11, 1987, 369-375.

La présence de disruption est vérifiée par PCR selon la technique de Gussow et al.: Direct Clone Characterization from Plaques and Colonies by the Polymerase Chain Reaction. Nucleic Acids Res. 17, 1989, 4000, puis confirmée par hybridation Southern blot.

Au départ de la souche Po1d, les disruptions sont effectuées en deux étapes.

Dans un premier temps, Po1d est transformé avec la cassette 1 de disruption de PCR PUT et sélectionné. Dans un deuxième temps, les clones Ura+ sont transformés avec la cassette de disruption 2, pour éliminer le gène URA3 et sont sélectionnés. Ce protocole permet l'obtention du quadruple disruptant MTLY37 - pox2ΔPT- pox3ΔPT- pox4ΔPT-pox5ΔPUT. La représentation schématique de la construction de ce mutant est résumée dans le Tableau 1 ci-dessous.

**Tableau 1 : Étapes de transformation requises pour la production du mutant MTLY37 ne poussant plus sur acide oléique**

| **Étape** | **Opérations de transformation** | | |
|---|---|---|---|
| | **Mutant à transformer** | **Cassette de transformation** | **Mutant transformé** |
| 1 | PO1d, Leu-, Ura- | POX5-PUT | MTLY15, Leu-, Ura+, Δ5-PUT |
| 2 | MTLY15, Leu-, Ura+, Δ5-PUT | LEU2 | MTLY19, Leu+, Ura+, Δ5-PUT |
| 3 | MTLY19, Leu+, Ura+, Δ5-PUT | POX5, PT | MTLY24, Leu+, Ura-, Δ5-PT |
| 4 | MTLY24, Leu+, Ura-, Δ5-PT | POX2, PUT | MTLY29, Leu+, Ura+, Δ5-PT, Δ2-PUT |
| 5 | MTLY29, Leu+, Ura+, Δ5-PT, Δ2-PUT | POX2-PT | MTLY32, Leu+, Ura-, Δ5-PT, Δ2-PT |
| 6 | MTLY32,Leu+, Ura-, Δ5-PT, Δ2-PT | POX3-PUT | MTLY35, Leu+, Ura+, Δ5-PT, Δ2-PT, Δ3-PUT |
| 7 | MTLY35,Leu+, Ura+, Δ5-PT, Δ2-PT, Δ3-PUT | POX3-PT | MTLY36, Leu+, Ura-, Δ5-PT, Δ2-PT, Δ3-PT |
| 8 | MTLY36, Leu+, Ura-, Δ5-PT, Δ2-PT, Δ3-PT | MTLY18 délété pour POX4 (Δ4-PUT) | MTLY37, Leu+, Ura+, Δ5-PT, Δ2-PT, Δ3-PT, Δ4-PUT |

La disruption d'un gène et l'excision du marqueur peuvent encore se faire par une méthode mettant en jeu une recombinaison ou une recombinase. On peut par exemple utiliser des marqueurs avec de part et d'autre une séquence répétée (permettant la recombinaison qui est sélectionnée) ou une séquence lox qui est reconnue par la recombinase Cre. L'excision a lieu quand on exprime la Recombinase Cre, Fickers et al., 2003 New Disruption Cassettes for Rapid Gene Disruption and Marker Rescue in the Yeast Yarrowia lipolytica. J. Microbiol. Methods 55/3:727-737.

### 2. Obtention de la souche Yarrowia lipolytica mutante MTLY74

À partir du mutant MTLY37, on a construit la souche MTLY74 Leu+Ura-.
**2a.** À partir du mutant MTLY37 prototrophe (Leu+, Ura+), on a construit une souche *Yarrowia lipolytica* mutante MTLY66 auxotrophe pour la leucine et l'uracile (Leu-, Ura-). La première étape est la construction de la souche MTLY40 auxotrophe pour l'uracile (Leu+, Ura-) par conversion du marqueur URA3 par le marqueur ura3-41 en transformant le fragment de PCR contenant ce marqueur et en sélectionnant les Ura- en présence de 5FOA. A partir du mutant MTLY40, on a construit une souche *Yarrowia lipolytica* mutante MTLY64 auxotrophe pour la leucine (Leu-, Ura-, Hyg+) par disruption du marqueur LEU2 en transformant la cassette de disruption PHTleu2 et en sélectionnant les transformants hygromycine résistant (leu2::Hyg). A partir du mutant MTLY64, on a construit une souche *Yarrowia lipolytica* mutante MTLY66 auxotrophe pour la leucine (Leu-, Ura-) par excision du marqueur HYG en transformant le vecteur réplicatif pRRQ2 contenant la recombinase Cre et le marqueur LEU2 (Cre-LEU2) et en sélectionnant les transformants hygromycine sensible, Leu+. La perte du plasmide pRRQ2 est réalisée par une culture sur milieu riche YPD et par l'isolement d'un clone (Leu-, Ura-, Hyg-).
**2b.** À partir du mutant MTLY66, on a construit une souche *Yarrowia lipolytica* mutante MTLY74 Leu+ Ura- qui surexprime la NADPH-cytochrome réductase, en l'exprimant sous le contrôle du promoteur fort pPOX2, induit par les substrats de bioconversion, du type acide gras, ester d'acide gras ou huile naturelle.

On a introduit le gène codant pour la NADPH-cytochrome réductase (*CPR*) dans un vecteur contenant le gène de sélection LEU2, par exemple JMP21, sous le contrôle du promoteur pPOX2 inductible par les acides gras, les esters d'acide gras ou les huiles naturelles. La cassette marqueur-promoteur-gène (LEU2-pPOX2-*CPR*) est introduite par transformation.

La représentation schématique de la construction de ce mutant est résumée dans le Tableau 2 ci-dessous.

**Tableau 2 : Étapes de transformation requises pour la production du mutant MTLY74 à partir de MTLY37**

| **Étape** | **Opérations de transformation** | | |
|---|---|---|---|
| | **Mutant à transformer** | **Transformation avec** | **Mutant transformé** |
| 1 | MTLY37, Leu+, Ura+, Δ5-PT, Δ2-PT, Δ3-PT, Δ4-PUT | Fragment de PCR ura3-41, sélection 5FOA | MTLY40, Leu+, Ura-, Δ5-PT, Δ2-PT, Δ3-PT, Δ4-Pura3-41T |
| 2 | MTLY40, Leu+, Ura-, Δ5-PT, Δ2-PT, Δ3-PT, Δ4-Pura3-41T | Cassette PHTleu2, sélection hygromycine | MTLY64, Leu-, Ura-, Hyg+, Δ5-PT, Δ2-PT, Δ3-PT, Δ4-Pura3-41T, Leu2::Hyg |
| 3 | MTLY64, Leu-, Ura-, Hyg+, Δ5-PT, Δ2-PT, Δ3-PT, Δ4-Pura3-41T, Leu2::Hyg | Vecteur pRRQ2, sélection Leu+, vérification Hyg-, perte du plasmide pRRQ2 sur YPD, isolement de Leu- | MTLY66, Leu-, Ura-, Δ5-PT, Δ2-PT, Δ3-PT, Δ4-Pura3-41T, Δleu2 |
| 4 | MTLY66, Leu-, Ura-, Δ5-PT, Δ-PT, Δ3-PT, Δ4-Pura3-41T, Δleu2 | Cassette d'expression pPOX2-CPR de JM21-*CPR* | MTLY74, Leu+, Ura-, Δ5-PT, Δ2-PT, Δ3-PT, Δ4-Pura3-41T, CPR |

### 3. Obtention des souches MTLY79, MTLY80 et MTLY81 à partir du mutant commun MTLY74

3a. MTLY79 exprimant la NADPH-cytochrome réductase et la cytochrome P450 monooxygénase *ALK1* sous le contrôle du promoteur pPOX2 inductible par les acides gras, les esters d'acide gras ou les huiles naturelles
À partir du mutant MTLY74, on a construit une souche *Yarrowia lipolytica* mutante MTLY79 qui surexprime la NADPH-cytochrome réductase et la cytochrome P450 monooxygénase *ALK*1 dans les conditions de bioconversion, sous le contrôle du promoteur fort pPOX2 induit par les substrats de bioconversion du type acide gras, ester d'acide gras ou huile naturelle.
On a introduit le gène *ALK*1 codant pour la cytochrome P450 monooxygénase dans un vecteur contenant le gène de sélection URA3, par exemple JMP61, sous le contrôle du promoteur pPOX2 inductible par les acides gras, les esters d'acide gras ou les huiles naturelles. La cassette marqueur-promoteur-gene (URA3-pPOX2-*AKL*1) est introduite par transformation.
La représentation schématique de la construction de ce mutant est résumée dans le Tableau 3 ci-dessous.

**Tableau 3 : Étape de transformation requise pour la production du mutant MTLY79 surexprimé pour ALK1 et CPR à partir de MTLY74**

| **Étape** | **Opération de transformation** | | |
|---|---|---|---|
| | **Mutant à transformer** | **Transformation avec** | **Mutant transformé** |
| 1 | MTLY74, Leu+, Ura-, Δ5-PT, Δ2-PT, Δ3-PT, Δ4-Pura3-41T, CPR | *JMP61-ALK1* | MTLY79, Leu+, Ura+, Δ5-PT, Δ2-PT, Δ3-PT, Δ4-Pura3-41T, *CPR, ALK*1 |

3b. MTLY80 exprimant la NADPH-cytochrome réductase et la cytochrome P450 monooxygénase *ALK2* sous le contrôle du promoteur pPOX2 inductible par les acides gras, les esters d'acide gras ou les huiles naturelles
À partir du mutant MTLY74, on a construit une souche *Yarrowia lipolytica* mutante MTLY80 qui surexprime les gènes codant pour la NADPH-cytochrome réductase (*CPR*) et la cytochrome P450 monooxygénase (*ALK2*) dans les conditions de bioconversion, sous le contrôle du promoteur fort pPOX2 induit par les substrats de bioconversion du type acide gras, ester d'acide gras ou huile naturelle.
On a introduit le gène *ALK2* codant pour la cytochrome P450 monooxygénase dans un vecteur contenant le gène de sélection URA3, par exemple JMP61, sous le contrôle du promoteur pPOX2 inductible par les acides gras, les esters d'acide gras ou les huiles naturelles. La cassette marqueur-promoteur-gène (URA3-pPOX2-*AKL2*) est introduite par transformation.
La représentation schématique de la construction de ce mutant est résumée dans le Tableau 4 ci-dessous.

**Tableau 4 : Étape de transformation requise pour la production du mutant MTLY80 surexprimé pour ALK2 et CPR à partir de MTLY74**

| **Étape** | **Opération de transformation** | | |
|---|---|---|---|
| | **Mutant à transformer** | **Transformation avec** | **Mutant transformé** |
| 1 | MTLY74, Leu+, Ura-, Δ5-PT, Δ2-PT, Δ3-PT, Δ4-Pura3-41T, *CPR* | JMP61-ALK2 | MTLY80, Leu+, Ura+, Δ5-PT, Δ2-PT, Δ3-PT, Δ4-Pura3-41T, *CPR, ALK*2 |

3c. MTLY81 exprimant la NADPH-cytochrome réductase sans les gènes de la cytochrome P450 monooxygénase (*ALK1* ou *ALK2*) sous le contrôle du promoteur pPOX2 inductible par les acides gras, les esters d'acide gras ou les huiles naturelles

À partir du mutant MTLY74, on a construit une souche *Yarrowia lipolytica* mutante MTLY81 qui surexprime le gène codant pour la NADPH-cytochrome réductase (*CPR*) sous le contrôle du promoteur fort pPOX2 induit par les substrats de bioconversion du type acide gras, ester d'acide gras ou huile naturelle.

On a rendu le mutant MTLY74 prototrophe par transformation avec le plasmide JMP61 portant le marqueur URA3.

La représentation schématique de la construction de ce mutant est résumée dans le Tableau 5 ci-dessous.

**Tableau 5 : Étape de transformation requise pour la production du mutant MTLY81 surexprimé pour CPR à partir de MTLY74**

| **Étape** | **Opérations de transformation** | | |
|---|---|---|---|
| | **Mutant à transformer** | **Transformation avec** | **Mutant transformé** |
| 1 | MTLY74, Leu+, Ura-, Δ5-PT, Δ2-PT, Δ3-PT, Δ4-Pura3-41T, *CPR* | JMP61 | MTLY81, Leu+, Ura+, Δ5-PT, Δ2-PT, Δ3-PT, Δ4-Pura3-41T, *CPR* |

### 4. Obtention de la souche mutante FT120

Pour obtenir cette souche, on peut procéder comme pour la construction de la souche MTLY37 ou de la souche MTLY66:
1) on construit une cassette de disruption par PCR ("Polymerase Chain Reaction") ou par clonage, en utilisant un marqueur contre-sélectionnable, par exemple le marqueur URA3 (avec lequel on peut sélectionner pour le phénotype Ura+ ou pour le phénotype Ura-), ou en utilisant un marqueur avec de part et d'autre une séquence répétée (permettant la recombinaison qui est sélectionnée) ou une séquence lox qui est reconnue par la recombinase Cre
2) on sélectionne les souches avec le gène d'intérêt délété (transformation et sélection des transformants ; avantageusement Ura+ si le marqueur est URA3) et on vérifie la disruption du gène ;
3) on sélectionne les souches avec le marqueur délété (transformation et sélection des transformants); avantageusement 5FOA^{R} si le marqueur est URA3 ou avantageusement un plasmide exprimant la recombinase si le marqueur présente la séquence lox et on vérifie la disruption du gène.

On décrit ci-après une méthode particulière d'obtention de la souche mutante FT120.

À partir du mutant MTLY66, on a construit la souche FT120 Leu- Ura-, Δpox1-6.
**4a.** À partir du mutant MTLY66 Δpox2-5, on a construit une souche *Yarrowia lipolytica* mutante MTLY95 Δpex1-6 par insertion de délétion des gènes POX1 et POX6 et délétion du marqueur selon la méthode décrite précédemment.
**4b.** À partir du mutant MTLY95, on a construit une souche *Yarrowia lipolytica* mutante FT101 Leu+ Ura- qui surexprime le gène codant pour la NADPH-cytochrome réductase (*CPR*) dans les conditions de bioconversion, en l'exprimant sous le contrôle, du promoteur fort pPOX2, induit par les substrats de bioconversion, du type acide gras, ester d'acide gras ou huile naturelle.

On a introduit le gène codant pour pour la NADPH-cytochrome réductase dans un vecteur contenant le gène de sélection LEU2 excisable, par exemple JMP21-LEU2ex, sous le contrôle du promoteur pPOX2 inductible par les acides gras, les esters d'acide gras ou les huiles naturelles. La cassette marqueur-promoteur-gène (LEU2ex-pPOX2-CPR) est introduite par transformation. La souche FT120 est obtenue à la suite de l'excision du marqueur LEU2ex par transformation avec le plasmide pUB4-CRE, sélection Hyg+, perte du plasmide sur YPD et enfin isolement d'un clone Leu-.

La représentation schématique de la construction de ce mutant est résumée dans le Tableau 6 ci-dessous.

**Tableau 6 : Étapes de transformation requises pour la production du mutant FT120 à partir de MTLY66**

| **Étape** | **Opérations de transformation** | | |
|---|---|---|---|
| | **Mutant à transformer** | **Cassette de transformation** | **Mutant transformé** |
| 1 | MTLY66, Leu-, Ura-, Hyg-, Δ5-PT, Δ2-PT, Δ3-PT, Δ4-Pura3-41T | POX1-PHT | MTLY82, Leu-, Ura-, Hyg+, Δ5-PT, Δ2-PT, Δ3-PT, Δ4-Pura3-41T , Δ1-PHT |
| 2 | MTLY82, Leu-, Ura-, Hyg+, Δ5-PT, Δ2-PT, Δ3-PT, Δ4-Pura3-41T, Δ1-PHT | Vecteur pRRQ2, sélection Leu+, vérification Hyg-, perte du plasmide pRRQ2 sur YPD, isolement de Leu- | MTLY85 Leu-, Ura-, Hyg-, Δ5-PT, Δ2-PT, Δ3-PT, Δ4-Pura3-41T , Δ1-PT |
| 3 | MTLY85 Leu-, Ura-, Hyg-, Δ5-PT, Δ12-PT, Δ3-PT, Δ4-Pura3-41T , Δ1-PT | POX6-PHT | MTLY92 Leu-, Ura-, Hyg+, Δ5-PT, Δ2-PT, Δ3-PT, Δ4-Pura3-41T , Δ1-PT, Δ6-PHT |
| 4 | MTLY92 Leu-, Ura-, Hyg+, Δ5-PT, Δ2-PT, Δ3-PT, Δ4-Pura3-41T, Δ1-PT, Δ6-PHT | Vecteur pRRQ2, sélection Leu+, vérification Hyg-, perte du plasmide pRRQ2 sur YPD, isolement de Leu- | MTLY95 Leu-, Ura-, Hyg-, Δ5-PT, Δ2-PT, Δ3-PT, Δ4-Pura3-41T, Δ1-PT, Δ6-PT |
| 5 | MTLY95 Leu-, Ura-, Hyg-, Δ5-PT, Δ2-PT, Δ3-PT, Δ4-Pura3-41T , Δ1-PT, Δ6-PT | Cassette d'expression pPOX2-CPR de JM21-LEU2ex-CPR | FT101, Leu+, Ura-, Hyg-, Δ5-PT, Δ2-PT, Δ3-PT, Δ4-Pura3-41T , Δ1-PT, Δ6-PT, CPR-LEU2ex |
| 6 | FT101, Leu+, Ura-, Hyg-, Δ5-PT, Δ2-PT, Δ3-PT, Δ4-Pura3-41T , Δ1-PT, Δ6-PT, CPR-LEU2ex | Vecteur pUB4-CRE, sélection Hyg+, vérification Leu-, perte du plasmide pUB4-CRE sur YPD, isolement de Hyg- | FT120, Leu-, Ura-, Hyg-, Δ5-PT, Δ2-PT, Δ3-PT, Δ4-Pura3-41T , Δ1-PT, Δ6-PT, CPR |

### 5. Obtention de la souche mutante FT130

Pour obtenir cette souche, on peut procéder comme pour la construction de la souche FT120 :
1) on construit une cassette de disruption par PCR ("Polymerase Chain Reaction") ou par clonage, en utilisant un marqueur contre-sélectionnable, par exemple le marqueur URA3 (avec lequel on peut sélectionner pour le phénotype Ura+ ou pour le phénotype Ura-), ou en utilisant un marqueur avec de part et d'autre une séquence répétée (permettant la recombinaison qui est sélectionnée) ou une séquence lox qui est reconnue par la recombinase Cre ;
2) on sélectionne les souches avec le gène d'intérêt délété (transformation et sélection des transformants ; avantageusement Ura+ si le marqueur est URA3) et on vérifie la disruption du gène ;
3) on sélectionne les souches avec le marqueur délété (transformation et sélection des transformants) ; avantageusement 5FOA^{R} si le marqueur est URA3 ou avantageusement un plasmide exprimant la recombinase Cre si le marqueur présente la séquence lox et on vérifie la disruption du gène.

On décrit ci-après une méthode particulière d'obtention de la souche mutante FT130.

À partir du mutant FT120, on a construit la souche FT130 Leu- Ura-, Δpex1-6, Δga1.

### 6. À partir du mutant FT120 Leu- Ura-, Δpox1-6, pPOX2-CPR on a construit une souche Yarrowia lipolytica mutante FT130 Leu-Ura+, Δpex1-6, pPOX2-CPR, Δdga1 par insertion de délétion du gène DGA1 codant pour l'acyl-CoA diacylglycerol acyltransferase.

**Tableau 7 : Étape de transformation requise pour la production du mutant FT130 à partir de FT120**

| **Étape** | **Opérations de transformation** | | |
|---|---|---|---|
| | **Mutant à transformer** | **Cassette de transformation** | **Mutant transformé** |
| 1 | FT120, Leu-, Ura-, Hyg-, Δ5-PT, Δ2-PT, Δ3-PT, Δ4-Pura3-41T, Δ1-PT,Δ6-PT, CPR | DGA1-PUT | FT130, Leu-, Ura+, Hyg-, Δ5-PT, Δ2-PT, Δ3-PT, Δ4-Pura3-41T , Δ1-PT, Δ6-PT, CPR, Δdga1-PUT |

Les souches MTLY66, MTLY81, FT120 et FT 130 ont été déposées à la Collection Nationale de Cultures de Microorganismes sous les numéros d'enregistrement respectifs CNCM I-3319, CNCM I-3320, CNCM I-3527 et CNCM I-3528.

Les exemples suivants illustrent l'invention sans en limiter la portée.

### EXEMPLES

Dans ces exemples, on a testé l'influence des conditions de culture et de la composition du milieu sur la production de diacides. On a ainsi constaté avec le mutant MTLY37 que l'utilisation de peptone favorisait fortement la production de diacides, notamment par rapport à la bacto-tryptone (Exemples 1 et 2).

On a en outre testé dans les mêmes conditions les mutants MTLY79, MTLY80 et MTLY81. On a ainsi constaté que la NADPH-cytochrome réductase catalysait une étape limitante dans la production de diacides. En effet, la surexpression de cette enzyme seule permet d'augmenter significativement la production et la productivité des diacides (Exemples 4 à 6).

On a testé dans des conditions identiques les souches mutantes FT120 et FT130 (exemples 7 et 8). La délétion des gènes POX1 et POX 6 permet de diminuer la dégradation des diacides pour FT120. La délétion d'un gène supplémentaire DGA1 codant pour l'acyl-CoA diacylglycerol acyltransferase entraîne une diminution de l'accumulation du substrat de bioconversion sous forme de corps lipidiques au sein de la cellule de *Yarrowia lipolytica.* Ainsi la majorité des diacides obtenus dans ces exemples est composée de diacides à 18 atomes de carbone à l'image du substrat de bioconversion utilisé, composé en majorité d'acides gras à 18 atomes de carbones.

### Exemple 1 : Procédé de production d'acides dicarboxyliques à partir d'huile de tournesol oléique avec le mutant MTLY37

Une préculture du mutant MTLY37, conservé sur milieu gélosé de composition : extrait de levure 10 g.l⁻¹; peptone 10 g.l⁻¹; glucose 10 g.l⁻¹ ; Agar 20 g.l⁻¹, est effectué grâce à un ensemencement qui fournit une absorbance initiale du milieu de préculture voisine de 0,30. La préculture est conduite sous agitation orbitale (200 rpm) pendant 24 h à 30°C dans une fiole à ailettes de 500 ml contenant 25 ml de milieu (10 g.l⁻¹ extrait de levure; 10 g.l⁻¹ peptone; 20 g.l⁻¹ glucose).

Le milieu utilisé pour la culture est composé d'eau désionisée, d'extrait de levure à 10 g.l⁻¹; de tryptone à 20 g.l⁻¹; de glucose à 40 g.1⁻¹; et d'huile de tournesol oléique à 30 g.l⁻¹.

L'ensemencement du fermenteur est effectué avec la totalité de la fiole de préculture.

La culture est conduite à 30°C dans un fermenteur de 4 I avec 2 I de milieu à un débit d'aération de 0,5 wm et une vitesse d'agitation de 800 rpm assurée par une turbine centripète à double effet.

Après 17 heures de culture dès lors que le glucose du milieu est épuisé, on ajoute 60 ml d'huile de tournesol oléique, composée en majorité d'acides gras à 18 atomes de carbone, dans le réacteur que l'on soumet à une alimentation continue de glycérol à raison de 1 ml.h⁻¹. Le pH de la culture est alors maintenu à la valeur constante de 8 par addition régulée de soude 4 M. La fermentation dure 130 h.

En fin de culture, on élimine la biomasse cellulaire par centrifugation. Le surnageant est ensuite acidifié jusqu'à pH 2,5 par addition d'HCl 6M et les acides dicarboxyliques insolubles sont récupérés par centrifugation du moût acidifié puis séchés.

La composition en acides dicarboxyliques du mélange est déterminée par chromatographie en phase gazeuse sur une colonne DB1 après conversion des acides dicarboxyliques en diesters selon la méthode décrite par Uchio et al.: Microbial Production of Long-chain Dicarboxylic Acids from n-Alkanes. Part Il. Production by Candida cloacae Mutant Unable to Assimilate Dicarboxylic Acid. Agr Biol. Chem. 36, No. 3, 1972, 426-433. La température du four du chromatographe est programmée de 150°C à 280°C à raison de 8°C par min.

Les résultats montrent une production maximale de diacides dans les surnageants de 5,9 g.l⁻¹ après 130 h.

### Exemple 2

On répète l'Exemple 1 en remplaçant dans le milieu de culture la tryptone par de la peptone à la même concentration. Au bout de 130 h de culture, on obtient 9,9 g.l⁻¹ d'acides dicarboxyliques, soit un accroissement de production d'environ 68 % par rapport à l'Exemple 1.

### Exemple 3 : Production d'acides dicarboxyliques par le mutant MTLY37 avec alimentation continue d'huile de tournesol oléique

On répète l'Exemple 2 en éliminant l'huile de tournesol oléique du milieu de culture et en la remplaçant par une injection continue de cette même huile à un flux sub-limitant de 1 ml dans le réacteur.

Dans ces conditions, 14,7 g.l⁻¹ d'acides dicarboxyliques sont produits dans le milieu de culture après 130 h.

### Exemple 4 : Production d'acides dicarboxyliques à partir d'huile de tournesol oléique avec le mutant MTLY79 surexprimé pour CPR et ALK1

On répète l'Exemple 3 en remplaçant le mutant MTLY37 par le mutant MTLY79 surexprimant les gènes *CPR* et *ALK1.* Au bout de 130 h de culture, on obtient 16 g.l⁻¹ d'acides dicarboxyliques.

### Exemple 5 : Production d'acides dicarboxyliques à partir d'huile de tournesol oléique avec le mutant MTLY80 surexprimé pour CPR et ALK2

On répète l'Exemple 3 en remplaçant le mutant MTLY37 par le mutant MTLY80 surexprimant les gènes *CPR* et *ALK2.* Au bout de 130 h de culture, on obtient 16 g.l⁻¹ d'acides dicarboxyliques.

### Exemple 6

On répète l'Exemple 3 en remplaçant le mutant MTLY37 par le mutant MTLY81 surexprimant uniquement le gène *CPR.* Au bout de 130 h de culture, on obtient 16 g.l⁻¹ d'acides dicarboxyliques.

### Exemple 7 : Production d'acides dicarboxyliques à partir d'huile de tournesol oléique avec le mutant FT120 délété pour les six gènes POX (Δpex1-6) et surexprimant le gène CPR

On répète l'Exemple 3 en remplaçant le mutant MTLY37 par le mutant FT120 délété des six gènes *POX* et surexprimant le gène *CPR.* Au bout de 130 h de culture, on obtient 18 g.l⁻¹ d'acides dicarboxyliques

### Exemple 8 : Production d'acides dicarboxyliques à partir d'huile de tournesol oléique avec le mutant FT130 délété pour les six gènes POX (Δpox1-6) et délété pour le gène DGA1 (Δga1) et surexprimant le gène CPR

On répète l'Exemple 3 en remplaçant le mutant MTLY37 par le mutant FT130 délété des six gènes *POX* et du gène *DGA*1 et surexprimant le gène *CPR.* Au bout de 130 h de culture, on obtient 23 g.l⁻¹ d'acides dicarboxyliques

## Revendications

1. Procédé de production d'acides dicarboxyliques, **caractérisé en ce qu'**il comprend :
a) une phase de croissance, dans laquelle on met en culture une souche mutante de *Yarrowia lipolytica* disruptée au moins pour les gènes codant pour les iso-enzymes de l'acyl-CoA oxydase *POX2, POX3, POX4* et *POX5;* dans un milieu de culture constitué essentiellement d'un substrat énergétique qui comprend au moins une source de carbone et une source d'azote ;
b) une phase de bioconversion, dans laquelle on soumet ladite souche à un substrat de bioconversion choisi parmi les n-alcanes d'au moins 10 atomes de carbone, les acides gras d'au moins 10 atomes de carbone, les esters d'alkyle de 1 à 4 atomes de carbone de ces acides gras et les huiles naturelles, en présence d'un substrat énergétique ; et
c) une phase de récupération de l'acide dicarboxylique formé.

2. Procédé selon la revendication 1 **caractérisé en ce que** le mutant utilisé est MTLY37, issu de la souche sauvage ATCC 20460, répertoriée sous CLIB89 dans la Collection de Levures d'Intérêt Biotechnologiques, et obtenu par les opérations de transformations suivantes à partir de la souche Po1d répertoriée sous CLIB139 dans la Collection de Levures d'Intérêt Biotechnologique,
| Étape | Opérations de transformation | | |
|---|---|---|---|
| | Mutant à transformer | Cassette de transformation | Mutant transformé |
| 1 | PO1d, Leu-, Ura- | POX5-PUT, le terme PUT correspondant au module promoteur-URA3-terminateur | MTLY15, Leu-, Ura+, Δ5-PUT |
| 2 | MTLY15. Leu-, Ura+, Δ5-PUT | LEU2 | MTLY19, Leu+, Ura+, Δ5-PUT |
| 3 | MTLY19, Leu+, Ura+, Δ5-PUT | POX5. PT, le terme PT correspondant au module promoteur-terminateur | MTLY24, Leu+, Ura-, Δ5-PT |
| 4 | MTLY24, Leu+, Ura-, Δ5-PT | POX2. PUT | MTLY29, Leu+, Ura+, Δ5-PT, Δ2-PUT |
| 5 | MTLY29, Leu+, Ura+, Δ5-PT, A2-PUT | POX2-PT | MTLY32, Leu+, Ura-, A5-PT, Δ2-PT |
| 6 | MTLY32,Leu+, Ura-, Δ5-PT, Δ2-PT | POX3-PUT | MTLY35, Leu+, Ura+, Δ5-PT, Δ2-PT, Δ3-PUT |
| 7 | MTLY35,Leu+, Ura+, Δ5-PT, Δ2-PT, Δ3-PUT | POX3-PT | MTLY36, Leu+, Ura-, Δ5-PT, Δ2-PT, Δ3-PT |
| 8 | MTLY36, Leu+, Ura-, Δ5-PT, Δ2-PT, Δ3-PT | MTLY18 délété pour POX4 (Δ4-PUT) | MTLY37, Leu+, Ura+, Δ5-PT, Δ2-PT, Δ3-PT, Δ4-PUT |

3. Procédé selon la revendication 1 **caractérisé en ce que** le mutant utilisé est MTLY79 issu de la souche sauvage ATCC 20460, répertoriée sous CLIB89 dans la Collection de Levures d'Intérêt Biotechnologiques, surexprimant les gènes CPR et *ALK*1 codant respectivement pour la NADPH-cytochrome réductase et la cytochrome P450 monooxygénase, et obtenu par les opérations de transformations suivantes à partir du mutant MTLY74, lui même obtenu à partir du mutant MTLY37 tel que décrit dans la revendication 2 selon les opérations de transformation ci-dessous:
| Étape | Opérations de transformation | | |
|---|---|---|---|
| | Mutant à transformer | Transformation avec | Mutant transformé |
| 1 | MTLY37, Leu+, Ura+, Δ5-PT, Δ2-PT, Δ3-PT, Δ4-PUT | Fragment de PCR ura3-41, sélection 5FOA | MTLY40, Leu+, Ura-, Δ5-PT, Δ2-PT, Δ3-PT, Δ4-Pura3-41T |
| 2 | MTLY40, Leu+, Ura-, Δ5-PT, Δ2-PT, Δ3-PT, Δ4-Pura3-41T | Cassette PHTleu2, sélection hygromycine | MTLY64, Leu-, Ura-, Hyg+, Δ5-PT, Δ2-PT, Δ3-PT, Δ4-Pura3-41T, Leu2::Hyg |
| 3 | MTLY64, Leu-, Ura-, Hyg+, Δ5-PT, Δ2-PT, Δ3-PT, Δ4-Pura3-41T, Leu2::Hyg | Vecteur pRRQ2, sélection Leu⁺, vérification Hyg-, perte du plasmide pRRQ2 sur YPD, isolement de Leu- | MTLY66, Leu-, Ura-, Δ5-PT, Δ2-PT. Δ3-PT, Δ4-Pura3-41T. Δleu2 |
| 4 | MTLY66, Leu-, Ura-, Δ5-PT, Δ2-PT, Δ3-PT, Δ4-Pura3-41T, Δleu2 | Cassette d'expression pPOX2-*CPR* de JM21-*CPR* | MTLY74, Leu+, Ura-, Δ5-PT, Δ2-PT, Δ3-PT, Δ4-Pura3-41T, *CPR* |
| Étape | Opération de transformation | | |
|---|---|---|---|
| | Mutant à transformer | Transformation avec | Mutant transformé |
| 1 | MTLY74, Leu+, Ura-, Δ5-PT, Δ2-PT, Δ3-PT, Δ4-Pura3-41T, CPR | JMP61-ALK1 | MTLY79, Leu+, Ura+, Δ5-PT, Δ2-PT, Δ3-PT, Δ4-Pura3-41T, *CPR, ALK*1 |

4. Procédé selon la revendication 1 **caractérisé en ce que** le mutant utilisé est MTLY80 issu de la souche sauvage ATCC 20460, répertoriée sous CLIB89 dans la Collection de Levures d'Intérêt Biotechnologiques, surexprimant les gènes *CPR* et *ALK2* codant respectivement pour la NADPH-cytochrome réductase et la cytochrome P450 monooxygénase, et obtenu par les opérations de transformations suivantes à partir du mutant MTLY74 tel que décrit dans la revendication 3
| *Étape* | Opération de transformation | | |
|---|---|---|---|
| | Mutant à transformer | Transformation avec | Mutant transformé |
| 1 | MTLY74, Leu+, Ura-, Δ5-PT, Δ2-PT, Δ3-PT, Δ4-Pura3-41T, *CPR* | JMP61-*ALK*2 | MTLY80, Leu+, Ura+ Δ5-PT, Δ2-PT, Δ3-PT, Δ4-Pura3-41T, *CPR, ALK*2 |

5. Procédé selon la revendication 1 **caractérisé en ce que** le mutant utilisé est MTLY81 issu de la souche sauvage ATCC 20460, répertoriée sous CLIB89 dans la Collection de Levures d'Intérêt Biotechnologiques, surexprimant le gène CPR codant pour la NADPH-cytochrome réductase, et obtenu par les opérations de transformations suivantes à partir du mutant MTLY74 tel que décrit dans la revendication 3
| Étape | Opérations de transformation | | |
|---|---|---|---|
| | Mutant à transformer | Transformation avec | Mutant transformé |
| 1 | MTLY74, Leu+, Ura-, Δ5-PT, Δ2-PT, Δ3-PT, Δ4-Pura3-41T, *CPR* | JMP61 | MTLY81, Leu+, Ura+, Δ5-PT, Δ2-PT, Δ3-PT, Δ4-Pura3-41T. *CPR* |

6. Procédé selon la revendication 1 **caractérisé en ce que** le mutant utilisé est FT 120 issu de la souche sauvage ATCC 20460, répertoriée sous CLIB89 dans la Collection de Levures d'Intérêt Biotechnologiques, surexprimant le gène CPR codant pour la NADPH-cytochrome réductase, et obtenu par les opérations de transformations suivantes à partir du mutant MTLY66 tel que décrit dans la revendication 3
| Étape | Opérations de transformation | | |
|---|---|---|---|
| | Mutant à transformer | Cassette de transformation | Mutant transformé |
| 1 | MTLY66, Leu-, Ura-, Hyg-, Δ5-PT, Δ2-PT, Δ3-PT, Δ4-Pura3-41T | POX1-PHT | MTLY82, Leu-, Ura-, Hyg+, Δ5-PT, Δ2-PT, Δ3-PT, Δ4-Pura3-41T , Δ1-PHT |
| 2 | MTLY82, Leu-, Ura-, Hyg+, Δ5-PT, Δ2-PT, Δ3-PT, Δ4-Pura3-41T , Δ1-PHT | Vecteur pRRQ2, sélection Leu+, vérification Hyg-, perte du plasmide pRRQ2 sur YPD, isolement de Leu- | MTLY85 Leu-, Ura-, Hyg-, Δ5-PT, Δ2-PT, Δ3-PT, Δ4-Pura3-41T , Δ1-PT |
| 3 | MTLY85 Leu-, Ura-, Hyg-, Δ5-PT, Δ2-PT, Δ3-PT, Δ4-Pura3-41T, Δ1-PT | POX6-PHT | MTLY92 Leu-, Ura-, Hyg+, Δ5-PT, Δ2-PT, Δ3-PT, Δ4-Pura3-41T, Δ1-PT, Δ6-PHT |
| 4 | MTLY92 Leu-, Ura-, Hyg+, Δ5-PT, Δ2-PT, Δ3-PT, Δ4-Pura3-41T, Δ1-PT, Δ6-PHT | Vecteur pRRQ2, sélection Leu+, vérification Hyg-, perte du plasmide pRRQ2 sur YPD, isolement de Leu- | MTLY95 Leu-, Ura-, Hyg-, Δ5-PT, Δ2-PT, Δ3-PT. Δ4-Pura3-41T, Δ1-PT, Δ6-PT |
| 5 | MOLY95 Leu-, Ura-, Hyg-, Δ5-PT, Δ2-PT, Δ3-PT, Δ4-Pura3-41T , Δ1-PT, Δ6-PT | Cassette d'expression pPOX2-CPR de JM21-LEU2ex-CPR | FT101, Leu+, Ura-, Hyg-, Δ5-PT, Δ2-PT, Δ3-PT, Δ4-Pura3-41T, Δ1-PT, Δ6-PT, CPR-LEU2ex |
| 6 | FT101, Leu+, Ura-, Hyg-, Δ5-PT, Δ2-PT, Δ3-PT, Δ4-Pura3-41T, Δ1-PT, Δ6-PT, CPR-LEU2ex | Vecteur pUB4-CRE, sélection Hyg+, vérification Leu-, perte du plasmide pUB4-CRE sur YPD, isolement de Hyg- | FT120, Leu-, Ura-, Hyg-, Δ5-PT, Δ2-PT, Δ3-PT, Δ4-Pura3-41T, Δ1-PT, Δ6-PT, CPR |

7. Procédé selon la revendication 1 **caractérisé en ce que** le mutant utilisé est FT 130 issu de la souche sauvage ATCC 20460, répertoriée sous CLIB89 dans la Collection de Levures d'Intérêt Biotechnologiques, surexprimant le gène CPR codant pour la NADPH-cytochrome réductase, et obtenu par les opérations de transformations suivantes à partir du mutant FT 120 tel que décrit dans la revendication 6
| Étape | Opérations de transformation | | |
|---|---|---|---|
| | Mutant à transformer | Cassette de transformation | Mutant transformé |
| 1 | FT120, Leu-, Ura-, Hyg-, Δ5-PT, Δ2-PT, Δ3-PT, Δ4-Pura3-41T, Δ1-PT, Δ6-PT, CPR | DGA1-PUT | FT130, Leu-, Ura+, Hyg-, Δ5-PT, Δ2-PT, Δ3-PT, Δ4-Pura3-41T, Δ1-PT, Δ6-PT, CPR, Δdga1-PUT |

8. Procédé selon l'une des revendications 1 à 5 **caractérisé en ce que** ledit substrat de bioconversion consiste en un mélange d'esters méthyliques ou un mélange d'esters éthyliques.

9. Procédé selon l'une des revendications 1 à 5 **caractérisé en ce que** ledit substrat de bioconversion consiste en une huile de tournesol oléique.

10. Procédé selon l'une des revendications 1 à 7 **caractérisé en ce que,** dans la phase de bioconversion, le milieu de culture comprend de la peptone.

11. Procédé selon l'une des revendications 1 à 8 **caractérisé en ce que**, dans la phase de bioconversion, le milieu de culture comprend un apport de substrat énergétique secondaire consistant en au moins un composé polyhydroxylé.

12. Procédé selon la revendication 9 **caractérisé en ce que** ledit composée polyhydroxylé est le glycérol ou un sucre.

13. Procédé selon l'une des revendications 1 à 10 **caractérisé en ce que**, dans la phase (c), l'acide dicarboxylique est récupéré par précipitation sous forme de sel de calcium.

14. Souche *Yarrowla lipolytica* mutante MTLY66 déposée à la Collection Nationale de Cultures de Microorganismes sous le numéro d'enregistrement CNCM 1-3319

15. Souche *Yarrowia lipolytica* mutante MTLY81 déposée à la Collection Nationale de Cultures de Microorganismes sous le numéro d'enregistrement CNCM 1-3320.

16. Souche *Yarrowia lipolytica* mutante FT120 déposée à la Collection Nationale de Cultures de Microorganismes sous le numéro d'enregistrement CNCM 1-3527.

17. Souche *Yarrowia lipolytica* mutante FT130 déposée à la Collection Nationale de Cultures de Microorganismes sous le numéro d'enregistrement CNCM 1-3528.

## Claims

1. A method of producing dicarboxylic acids, comprising:
(a) a growth stage wherein a mutant strain of *Yarrowia lipolylica* disrupted at least for the *POX2, POX3, POX4* and *POX5* genes coding for acyl-CoA oxidase is cultured in a culture medium essentially consisting of an energetic substrate comprising at least a source of carbon and a source of nitxogen,
(b) a bioconversion stage wherein said strain is subjected to a bioconversion substrate selected from among the *n*-alkanes having at least 10 carbon atoms, fatty acids having at least 10 carbon atoms, alkyl esters having 1 to 4 carbon atoms of these fatty acids and natural oils, in the presence of an energetic substrate, and
(c) a stage of recovering the dicarboxylic acid formed.

2. A method as claimed in claim 1, **characterized in that** the mutant used is MTLY37, derived from ATCC 20460 wild strain recorded under CLIB89 in the Collection de Levures d'Intérêt Biotcchnologique-CLIB and obtained by the following conversion operations from the Pold strain recorded under CLIB139 in the Collection de Levures d'Intérêt Bioteclnologique-CLIB
| **Stage** | **Conversion operations** | | |
|---|---|---|---|
| | **Mutant to be converted** | **Conversion cassette** | **Converted mutant** |
| 1 | POld,Leu-,Ura- | POX5-PUT | MTLY15, Leu-, Ura+, Δ5-PUT |
| 2 | MTLY15, Leu-, Ura+, Δ5-PUT | LEU2 | MTLY19, Leu+, Ura+, Δ5-PUT |
| 3 | MTLY19, Leu+, Ura+, Δ5-PUT | POX5, PT | MTLY24, Leu+, Ura-, Δ5-PT |
| 4 | MTLY24, Leu+, Ura-, Δ5-PT | POX2, PUT | MTLY29, Leu+, Ura+, Δ5-PT Δ2-PUT |
| 5 | MTLY29, Leu+, Ura+, Δ5-PT, Δ2-PUT | POX2-PT | MTLY32, Leu+, Ura-, Δ5-PT, Δ2-PT |
| 6 | MTLY32,Leu+, Ura-, Δ5-PT, Δ2-PT | POX3-PUT | MTLY35, Leu+, Ura+, Δ5-PT, Δ2-PT, Δ3-PUT |
| 7 | MTLY35,Leu+, Ura+, Δ5-PT, Δ2-PT, Δ3-PUT | POX3-PT | MTLY36, Leu+, Ura-, A5-PT, Δ2-PT, Δ3-PT |
| 8 | MTLY36, Leu+, Ura-, Δ5-PT, Δ2-PT, Δ3-PT | MTLY18 deleted for POX4 (Δ4-PUT) | MTLY37, Leu+, Ura+, Δ5-PT, Δ2-PT, Δ3-PT, Δ4-PUT |

3. A method as claimed in claim 1, **characterized in that** the mutant used is MTLY79 derived from ATCC 20460 wild strain recorded under CLIB89 in the Collection de Levures d'lntérêt Biotechnologiquc-CLIB overexpressing the *CPR* and *ALK1* genes respectively coding for the NADPH-cytochrome reductase and the cytochrome P450 monooxygenase, and obtained by the following conversion operations from MTLY74 mutant, itself obtained from the MTLY37 as described in claim 2 by the following conversion operations :
| **Stage** | **Conversion operations** | | |
|---|---|---|---|
| | **Mutant to be converted** | **Conversion with** | **Converted mutant** |
| 1 | MTLY37, Leu+, Ura+, Δ5-PT, Δ2-PT, Δ3-PT, Δ4-PUT | Fragment of PCR ura3-41, 5FOA selection | MTLY40, Leu+, Ura-, Δ5-PT, Δ2-PT, Δ3-PT, Δ4-Pura3-41 T |
| 2 | MTLY40, Leu+, Ura-, Δ5-PT, Δ2-PT, Δ3-PT, Δ4-Pura3-41T | PHTleu2 cassette, hygromycin selection | MTLY64, Leu-, Ura-, Hyg+, Δ5-PT, Δ2-PT, Δ3-PT, Δ4-Pura3-41T, Leu2::Hyg |
| 3 | MTLY64, Leu-, Ura-, Hyg+, Δ5-PT, Δ-PT, Δ3-PT, Δ4-Pura3-41T, Leu2::Hyg | pRRQ2 vector, Leu+ selection, checking Hyg-, loss of plasmid pRRQ2 on YPD, isolation of Leu- | MTLY66, Leu-, Ura-, Δ5-PT Δ2-PT, Δ3-PT, Δ4-Pura3-41T, Δleu2 |
| 4 | MTLY66, Leu-, Ura-, Δ5-PT, Δ2-PT, Δ3-PT, Δ4-Pura3-41T, Δleu2 | Expression cassette pPOX2-*CPR* of JM21-*CPR* | MTLY74, Leu+, Ura-, Δ5-PT, Δ2-PT, Δ3-PT, Δ4-Pura3-41T, *CPR,* |
| **Stage** | **Conversion operation** | | |
|---|---|---|---|
| | **Mutant to be converted** | **Conversion with** | **Converted mutant** |
| 1 | MTLY74, Leu+, Ura-, Δ5-PT, Δ2-PT, Δ3-PT, Δ4-Pura3-41T, CPR | *JMP61-ALK1* | MTLY79, Leu+, Ura+, Δ5-PT, Δ2-PT, Δ3-PT, Δ4-Pura3-41T, CPR, *ALK*1 |

4. A method as claimed in claim 1, **characterized in that** the mutant used is MTLY80 derived from ATCC 20460 wild strain recorded under CLIB89 in the Collection de Levures d'lntérêt Biotcdinologique-CLIB overexpressing the *CPR* and *ALK2* genes respectively coding for the NADPH-cytochrome reductase and the cytochrome P450 monooxygenase, and obtained by the following conversion operations from MTLY74 mutant, as described in claim 3 :
| **Stage** | **Conversion operation** | | |
|---|---|---|---|
| | **Mutant to be converted** | **Conversion with** | **Converted mutant** |
| 1 | MTLY74, Leu+, Ura-, Δ5-PT, Δ2-PT, Δ3-PT, Δ4-Pura3-41T, *CPR* | JMP61-ALK2 | MTLY80, Leu+, Ura+, Δ5-PT, Δ2-PT, Δ3-PT, Δ4-Pura3-41T, *CPR*, *ALK2* |

5. A method as claimed in claim 1, **characterized in that** the mutant used is MTLY81 derived from ATCC 20460 wild strain recorded under CLIB89 in the Collection de Levures d'Intérêt Biotcchnologique-CLIB overexpressing the *CPR* gene coding for the NADPH-cytochrome reductase, and obtained by the following conversion operations from MTLY74 mutant, as described in claim 3 :
| **Stage** | **Conversion operations** | | |
|---|---|---|---|
| | **Mutant to be converted** | **Conversion with** | **Converted mutant** |
| 1 | MTLY74, Leu+, Ura-, Δ5-PT, Δ2-PT, Δ3-PT, Δ4-Pura3-41T, CPR | JMP61 | MTLY81, Leu+, Ura+, Δ5-PT, Δ2-PT, Δ3-PT, Δ4-Pura3-41T, CPR |

6. A method as claimed in claim 1, **characterized in that** the mutant used is FT120 derived from ATCC 20460 wild strain recorded under CLIB89 in the Collection de Levures d'Intérêt Biotcchnologique-CLIB overexpressing the CPR gene coding for the NADPH-cytochrome reductase, and obtained by the following conversion operations from MTLY66 mutant, as described in claim 3
| **Stage** | **Conversion operations** | | |
|---|---|---|---|
| | **Mutant to be converted** | **Conversion cassette** | **Converted mutant** |
| 1 | MTLY66, Leu-, Ura-, Hyg-, Δ5-PT, Δ2-PT, Δ3-PT, Δ4-Pura3-41T | POX1-PHT | MTLY82, Leu-, Ura-, Hyg+, Δ5-PT, Δ2-PT, Δ3-PT, Δ4-Pura3-41T, Δ1-PHT |
| 2 | MTLY82. Leu-, Ura-, Hyg+, Δ5-PT, Δ2-PT, Δ3-PT, Δ-Pura3-41T , Δ1-PHT | pRRQ2 vector, Leu+ selection, checking Hyg-, loss of plasmid pRRQ2 on YPD, isolation of Leu- | MTLY85 Leu-, Ura-, Hyg-, Δ5-PT, Δ2-PT, Δ3-PT, Δ4-Pura3-41T , Δ1-PT |
| 3 | MTLY85 Leu-, Ura-, Hyg-, Δ5-PT, Δ2-PT, Δ3-PT, Δ4-Pura3-41T , Δ1-PT | POX6-PHT | MTLY92 Leu-, Ura-, Hyg+, Δ5-PT, Δ2-PT, Δ3-PT, Δ4-Pura3-41T, Δ1-PT, Δ6-PHT |
| 4 | MTLY92 Leu-, Ura-, Hyg+, Δ5-PT, Δ2-PT, Δ3-PT, Δ4-Pura3-41T, Δ1-PT, Δ6-PHT | pRRQ2 vector, Leu+ selection, checking Hyg-, loss of plasmid pRRQ2 on YPD, isolation of Leu- | MTLY95 Leu-, Ura-, Hyg-, Δ5-PT, Δ2-PT, Δ3-PT, Δ4-Pura3-41T, Δ1-PT, Δ6-PT |
| 5 | MTLY95 Leu-, Ura-, Hyg-. Δ5-PT. Δ2-PT, Δ-PT, Δ4-Pura3-41T, Δ1-PT, Δ6-PT | Expression cassette pPOX2-CPR of JM21-LEU2ex-CPR | FT101, Leu+, Ura-, Hyg-, Δ-PT, Δ2-PT, Δ3-PT, Δ4-Pura3-41T, Δ1-PT, Δ6-PT, CPR-LEU2ex |
| 6 | FT101, Leu+, Ura-, Hyg-, Δ5-PT, Δ2-PT, Δ3-PT, Δ4-Pura3-41T , Δ1-PT, Δ6-PT, CPR-LEU2ex | pUB4-CRE vector, Hyg+ selection, checking Leu-, loss of plasmid pUB4-CRE on YPD, isolation of Hyg- | FT120, Leu-, Ura-, Hyg-, Δ5-PT, Δ2-PT, Δ3-PT, Δ4-Pura3-41T , Δ1-PT, Δ6-PT, CPR |

7. A method as claimed in claim 1, **characterized in that** the mutant used is FT130 derived from ATCC 20460 wild strain recorded under CLIB89 in the Collection de Levures d'Intérêt Biotechnologique-CLIB overexpressing the *CPR* gene coding for the NADPH-eytochrome reductase, and obtained by the following conversion operations from FT120 mutant, as described in claim 6.
| **Stage** | **Conversion operations** | | |
|---|---|---|---|
| | **Mutant to be converted** | **Conversion cassette** | **Converted mutant** |
| 1 | FT120, Leu-, Ura-, Hyg-, Δ5-PT, Δ2-PT, Δ3-PT, Δ4-Pura3-41T , Δ1-PT, Δ6-PT, CPR | DGA1-PUT | FT130, Leu-, Ura+, Hyg-, Δ5-PT, Δ2-PT, Δ3-PT, Δ-Pura3-41T, Δ1-PT, Δ6-PT, CPR, Δdga1-PUT |

8. A method as claimed in any one of claims I to 5, **characterized in that** said bioconversion substrate consists of a mixture of methyl esters or of a mixture of ethyl esters.

9. A method as claimed in any one of claims 1 to 5, **characterized in that** said bioconversion substrate consists of an oleic sunflower oil.

10. A method as claimed in any one of stages 1 to 7, **characterized in that**, in the bioconversion stage, the culture medium comprises peptone.

11. A method as claimed in any one of claims 1 to 8, **characterized in that**, in the bioconversion stage, the culture medium comprises a supply of secondary energetic substrate consisting of at least one polyhydroxyl compound.

12. A method as claimed in claim 9, **characterized in that** said polyhydroxyl compound is glycerol or a sugar.

13. A method as claimed in any one of claims I to 10, **characterized in that**, in stage (c), the dicarboxylic acid is recovered by precipitation in form of calcium salt.

14. A mutant *Yarrowia lipolytica* strain MTLY66 registered at the Collection Nationale de Cultures de Microorganismes under the registration numbers CNCM 1-3319.

15. A mutant *Yarrowia lipolytica* strain MTLY81 registered at the Collection Nationale de Cultures de Microorganismes under the registration numbers CNCM I-3320

16. A mutant *Yarrowia lipolytica* strain FT120 registered at the Collection Nationale de Cultures de Microorganismes under the registration numbers CNCM I-3527

17. A mutant *Yarrowia lipolytica* strain FT130 registered at the Collection Nationale dc Cultures de Microorganismes under the registration numbers CNCM I-3528.

## Patentansprüche

1. Verfahren zur Herstellung von Dicarbonsäuren, **dadurch gekennzeichnet, dass** es umfasst:
a) eine Wachstumsphase, in der ein mutierter *Yarrowia-lipolyfica-*Stamm, der für mindestens die Gene disruptiert ist, die für die Isoenzyme der Acyl-CoA-Oxydase *POX2, POX3, POX4* und *POX5* kodieren, in einem Kulturmedium kultiviert wird, das im Wesentlichen aus einem energiereichen Substrat besteht, das mindestens eine Kohlenstoffquelle und eine Stickstoffquelle umfasst;
b) eine Biokonversionsphase, in der der Stamm einem Biokonversionssubstrat, das aus den *n*-Alkanen mit mindestens 10 Kohlenstoffatomen, den Fettsäuren mit mindestens 10 Kohlenstoffatomen, den Alkylestern mit 1 bis 4 Kohlenstoffatomen dieser Fettsäuren und den natürlichen Ölen ausgewählt ist, in Gegenwart eines energiereichen Substrats ausgesetzt wird; und
c) eine Phase der Gewinnung der gebildeten Dicarbonsäure.

2. Verfahren nach Anspruch 1, **dadurch gekennzeichnet, dass** der verwendete Mutant MTLY37 ist, der aus dem wilden Stamm ATCC 20460 stammt, der unter CLIB89 in die Collection de Levures d'Intérêt Biotechnologiques (Sammlung von Hefen von biotechnologischem Interesse) aufgenommen wurde, und durch die folgenden Transformationsvorgänge aus dem Stamm Po1d erhalten wurde, der unter CLIB139 in die Collection de Levures d'Intérêt Biotechnologique aufgenommen wurde.
| Schritt | Transformationsvorgänge | | |
|---|---|---|---|
| | zu transformierender Mutant | Transformationsvorgänge | transformierter Mutant |
| 1 | PO1d, Leu-, Ura- | POX5-PUT, wobei der Begriff PUT dem Modul Promoter-URA3-Terminator entspricht | MTLY15, Leu-, Ura+, Δ5-PUT |
| 2 | MTLY15, Leu-, Ura+, Δ5-PUT | LEU2 | MTLY19, Leu+, Ura+, Δ5-PUT |
| 3 | MTLY19, Leu+, Ura+, Δ5-PUT | POX5-PT, wobei der Begriff PT dem Modul Promoter-Terminator entspricht | MTLY24, Leu+, Ura-, Δ5-PT |
| 4 | MTLY24, Leu+, Ura-, Δ5-PT | POX2, PUT | MTLY29, Leu+, Ura+, Δ5-PT, Δ2-PUT |
| 5 | MTLY29, Leu+, Ura+, Δ5-PT, Δ2-PUT | POX2-PT | MTLY32, Leu+, Ura-, Δ5-PT, Δ2-PT |
| 6 | MTLY32, Leu+, Ura-, Δ5-PT, Δ-PT | POX3-PUT | MTLY35, Leu+, Ura+, Δ5-PT, Δ2-PT, Δ3-PUT |
| 7 | MTLY35, Leu+, Ura+, Δ5-PT, Δ2-PT, Δ3-PUT | POX3-PT | MTLY36, Leu+, Ura-, A5-PT, Δ2-PT, Δ-PT |
| 8 | MTLY36, Leu+, Ura-, Δ5-PT, Δ2-PT, Δ3-PT | MTLY18, das für POX4 deletiert ist (Δ4-PUT) | MTLY37, Leu+, Ura+, Δ5-PT, Δ2-PT, Δ3-PT, Δ4-PUT |

3. Verfahren nach Anspruch 1, **dadurch gekennzeichnet, dass** der verwendete Mutant MTLY79 ist, der aus dem wilden Stamm ATCC 20460 stammt, der unter CLIB89 in die Collection de Levures d'Interêt Biotechnologiques aufgenommen wurde, der die Gene CPR und *ALK1* überexprimiert, die jeweils für die NADPH-Cytochromreductase und die Cytochrom-P450-Monooxygease kodieren, und durch die folgenden Transformationsvorgänge aus dem Mutanten MTLY7A erhalten wird, der seinerseits aus dem Mutanten MTLY37, wie in Anspruch 2 beschrieben, gemäß den nachfolgenden Transformationsvorgängen erhalten wurde:
| Schritt | Transformationsvorgänge | | |
|---|---|---|---|
| | zu transformierender Mutant | Transformation mit | transformierter Mutant |
| 1 | MTLY37, Leu+, Ura+, Δ5-PT, Δ2-PT, Δ3-PT, Δ4-PUT | PCR-Fragment ura3-41, 5FOA-Selektion | MTLY40, Leu+, Ura-, Δ5-PT, Δ2-PT, Δ3-PT, Δ4- |
| 2 | MTLY40, Leu+, Ura-, Δ5-PT, Δ2-PT, Δ3-PT, Δ4-Pura3-41T | Kasette PHTleu2, Hygromycin-Selektion | MTLY64, Leu-, Ura-, Hyg+, Δ5-PT, Δ2-PT, Δ3-PT, Δ4-Pura3-41T, Leu2::Hyg |
| 3 | MTLY64, Leu-, Ura-, Hyg+, Δ5-PT, Δ2-PT, Δ3-PT, Δ4-Pura3-41T, Leu2::Hyg | Vektor pRRY2, Leu+-Selektion, Hyg--Verifikation, Teile des Plasmids pRRQ2 auf YPD, Isolation von Leu- | MTLY66, Leu-, Ura-, Δ5-PT, Δ2-PT, Δ3-PT, Δ4-Pura3-41T, Δleu2 |
| 4 | MTLY66, Leu-, Ura-, Δ5-PT, Δ2-PT, Δ3-PT, Δ4-Pura3-41T, Δleu2 | Expressionskassette pPOX2-CPR von JM21-CPR | MTLY74, Leu+, Ura-, Δ5-PT, Δ2-PT, Δ3-PT, Δ4-Pura3-41T, CPR |
| 1 | MTLY74, Leu+, Ura-, Δ5-PT, Δ2-PT, Δ3-PT, Δ4-Pura3-41T, CPR | JMP61-*ALK*1 | MTLY79, Leu+, Ura+, Δ5-PT, Δ2-PT, Δ3-PT, Δ4-Pura3-41T, CPR, *ALK*1 |

4. Verfahren nach Anspruch 1, **dadurch gekennzeichnet, dass** der verwendete Mutant MTLY80 ist, der aus dem wilden Stamm ATCC 20460 stammt, der unter CLIB89 in die Collection de Levures d'Interêt Biotechnologiques aufgenommen wurde, der die Gene CPR und *ALK*1 überexprimiert, die jeweils für die NADPH-Cytochromreductase und die Cytochrom-P450-Monooxygease kodieren, und durch die folgenden Transformationsvorgänge aus dem Mutanten MTLY74 erhalten wurde, wie in Anspruch 3 beschrieben:
| Schritt | Transformationsvorgänge | | |
|---|---|---|---|
| | zu transformierender Mutant | Transformation mit | transformierter Mutant |
| 1 | MTLY74, Leu+, Ura-, Δ5-PT, Δ2-PT, Δ3-PT, Δ4-Pura3-41T, CPR | JMP61-ALK2 *ALK2* | MTLY80, Leu+, Ura+, Δ5-PT, Δ2-PT, Δ3-PT, Δ4-Pura3-41T, CPR, |

5. Verfahren nach Anspruch 1, **dadurch gekennzeichnet, dass** der verwendete Mutant MTLY81 ist, der aus dem wilden Stamm ATCC 20460 stammt, der unter CLIB89 in die Collection de Levures d'Interêt Biotechnologiques aufgenommen wurde, der das Gen CPR überexprimiert, das für die NADPH-Cytochromreductase kodiert, und durch die folgenden Transformationsvorgänge aus dem Mutanten MTLY74 erhalten wurde, wie in Anspruch 3 beschrieben.
| Schritt | Transformationsvorgänge | | |
|---|---|---|---|
| | zu transformierender Mutant | Transformation mit | transformierter Mutant |
| 1 | MTLY74, Leu+, Ura-, Δ5-PT, Δ2-PT, Δ3-PT, Δ4-Pura3-41T, CPR | JMP61 | MTLY81, Leu+, Ura+, Δ5-PT, Δ2-PT, Δ3-PT, Δ4-Pura3-41T, CPR |

6. Verfahren nach Anspruch 1, **dadurch gekennzeichnet, dass** der verwendete Mutant FT 120 ist, der aus dem wilden Stamm ATCC 20460 stammt, der unter CLIB89 in die Collection de Levures d'Interêt Biotechnologiques aufgenommen wurde, der das Gen CPR überexprimiert, das für die NADPH-CytoChromreductase kodiert, und durch die folgenden Transformationsvorgänge aus dem Mutanten MTLY66 erhalten wurde, wie in Anspruch 3 beschrieben.
| Schritt | Transformationsvorgänge | | |
|---|---|---|---|
| | zu transformierender Mutant | Transformation mit | transformierter Mutant |
| 1 | MTLY66, Leu-, Ura-, Hyg-, Δ5-PT, Δ2-PT, Δ3-PT, Δ4-Pura3-41T | POX1-PHT | MTLY82, Leu-, Ura-, Hyg+, Δ5-PT, Δ2-PT, Δ3-PT, Δ4-Pura3-41T, Δ1-PHT |
| 2 | MTLY82, Leu-, Ura-, Hyg+, Δ5-PT, Δ2-PT, Δ3-PT, Δ4-Pur3-41T, Δ1-PHT | Vektor pRRQ2, Leu+-Selektion, Hyg-Verifikation, Verlust des Plasmids pRRQ2 auf YPD, Isolation von Leu- | MTLY85 Leu-, Ura-, Hyg-, Δ5-PT, Δ2-PT, Δ3-PT, Δ4-Pura3-41T, Δ1-PHT |
| 3 | MTLY85 Leu-, Ura-, Hyg-, Δ5-PT, Δ2-PT, Δ3-PT, A4-Pura3-41T, Δ1-PHT | POX6-PHT | MTLY92 Leu-, Ura-, Hyg+, Δ5-PT, Δ2-PT, Δ-PT, Δ4-Pura3-41T, Δ1-PT, Δ6-PHT |
| 4 | MTLY92 Leu-, Ura-, Hyg+, Δ5-PT, Δ2-PT, Δ3-PT, Δ-Pura3-41T, Δ1-PT, Δ6-PHT | Vektor pRRQ2, Leu+-Selektion, Hyg-Verifikation, Verlust des Plasmids pRRQ2 auf YPD, Isolation von Leu- | MTLY95 Leu-, Ura-, Hyg+, Δ5-PT, Δ2-PT, Δ-PT, Δ4-Pura3-41T, Δ1-PT, Δ6-PT |
| 5 | MTLY95 Leu-, Ura-, Hyg-, Δ5-PT, Δ2-PT, Δ3-PT, Δ4-Pura3-41T, Δ1-PT, Δ6-PT | Expressionskassette pPOX2-CPR von JM21-LEU2ex-CPR | FT101, Leu+, Ura-, Hyg-, Δ5-PT, Δ2-PT, Δ3-PT, Δ4-Pura3-41T, Δ1-PT, Δ6-PT, CPR-LEU2ex |
| 6 | FT101, Leu+, Ura-, Hyg-, Δ5-PT, Δ2-PT, Δ3-PT, Δ4-Pura3-41T, Δ1-PT, Δ6-PT, CPR-LEU2ex | Vektor pUB4-CRE, Hyg+-Selektion, Leu--Verifikation, Verlust des Plasmids pUB4-CRE auf YPD, Isolation von Hyg- | FT120, Leu-, Ura-, Hyg-, Δ5-PT, Δ2-PT, Δ3-PT, Δ4-Pura3-41T, Δ1-PT, Δ6-PT, CPR |

7. Verfahren nach Anspruch 1, **dadurch gekennzeichnet, dass** der verwendete Mutant FT 130 ist, der aus dem wilden Stamm ATCC 20460 stammt, der unter CLIB89 in die Collection de Levures d'Interêt Biotechnologiques aufgenommen wurde, der das Gen CPR überexprimiert, das für die NADPH-Cytochromreductase kodiert, und durch die folgenden Transformationsvorgänge aus dem Mutanten FT 120 erhalten wurde, wie in Anspruch 6 beschrieben.
| Schritt | Transformationsvorgänge | | |
|---|---|---|---|
| | zu transformierender Mutant | Transformationsvorgänge | transformierter Mutant |
| 1 | FT120, Leu-, Ura-, Hyg-, Δ5-PT, Δ2-PT, Δ3-PT, Δ4-Pura3-41T, Δ1-PT, Δ6a-PT, CPR | DGA1-PUT | FT130, Leu-, Ura+, Hyg-, Δ5-PT, Δ2-PT, Δ3-PT, Δ-Pura3-41T, Δ1-PT, Δ8-PT, CPR, |
| | | | Δdge1-PUT |

8. Verfahren nach einem der Ansprüche 1 bis 5, **dadurch gekennzeichnet, dass** das Biokonversionssubstrats aus einem Methylestergemisch oder einem Ethylestergemisch besteht.

9. Verfahren nach einem der Ansprüche 1 bis 5, **dadurch gekennzeichnet, dass** das Biokonversionssubstrat aus einem oleinischen Sonnenblumenöl besteht.

10. Verfahren nach einem der Ansprüche 1 bis 7, **dadurch gekennzeichnet, dass** das Kulturmedium in der Biokonversionsphase Pepton umfasst.

11. Verfahren nach einem der Ansprüche 1 bis 8, **dadurch gekennzeichnet, dass** das Kulturmedium in der Biokonversionsphase eine Zufuhr an sekundärem energiereichem Substrat umfasst, das aus mindestens einer polyhydroxylierten Verbindung besteht.

12. Verfahren nach Anspruch 9, **dadurch gekennzeichnet, dass** die polyhydroxylierte Verbindung Glycerol oder Zucker ist.

13. Verfahren nach einem der Ansprüche 1 bis 10, **dadurch gekennzeichnet, dass** in der Phase (c) die Dicarbonsäure durch Präzipitation in Form von Calciumsalz gewonnen wird.

14. Mutierter *Yarrowia-lipolytica-*Stamm MTLY66, der in der Collection nationale de Cultures de Microorganismes unter der Zugangsnummer CNCM I-3319 hinterlegt ist.

15. Mutierter *Yarrowia-lipolytica-*Stamm MTLY81, der in der Collection nationale de Cultures de Microorganismes unter der Zugangsnummer CNCM I-3320 hinterlegt ist.

16. Mutierter *Yarrowia-lipolytica-*Stamm FT120, der in der Collection nationale de Cultures de Microorganismes unter der Zugangsnummer CNCM I-3527 hinterlegt ist.

17. Mutierter *Yarrowia-lipolytica*-Stamm FT130, der in der Collection nationale de Cultures de Microorganismes unter der Zugangsnummer CNCM 1-3528 hinterlegt ist.
